# EUROPÄISCHE PATENTANMELDUNG

(11) **EP 2 842 600 A1**
(43) Veröffentlichungstag der Anmeldung: **04.03.2015**
(21) Anmeldenummer: 14174638.8
(22) Anmeldetag: 27.06.2014
(51) Int. Cl.: A61N 1/37

(54) **Implantierbares medizinisches Gerät und Verfahren zur Überwachung der Isolation einer Elektrodenleitung eines solchen medizinischen Gerätes**

(30) Priorität: 29.08.2013 US 201361871318 P
(71) Anmelder: BIOTRONIK SE & Co. KG, 12359 Berlin (DE)
(72) Erfinder: Dörr, Thomas, 12437 Berlin (DE)
(74) Vertreter: Lindner-Vogt, Karin L.

(57) **Zusammenfassung**

Ein implantierbares medizinisches Gerät, insbesondere aktives elektronisches Implantat, umfasst
- mindestens eine Elektrodenleitung (110) mit einem Elektrodenpol (130), einer Elektrodenzuleitung (120) und einer diese umgebenden Isolationshülle (125) gegenüber einem insbesondere durch Körperflüssigkeit gebildeten Elektrolyten (160),
- eine Gegenelektrode (140) zu der Elektrodenleitung (110),
- bezogen auf die elektrochemische Spannungsreihe unterschiedliche Materialien von Elektrodenzuleitung (120) und Elektrodenpol (130), und
- eine Isolationsprüfeinrichtung (50) mit einem zwischen Elektrodenpol (130) und Gegenelektrode (140) geschalteten Gleichspannungsdetektor (150) zur Erkennung einer bei einem Isolationsfehler der Isolationshülle (125) aufgrund des Kontaktes zwischen dem Elektrolyten (160) und der Elektrodenzuleitung (120) entstehenden elektrochemischen Spannung (V).

## Beschreibung

Die Erfindung betrifft ein implantierbares medizinisches Gerät umfassend mindestens eine Elektrodenleitung mit einem Elektrodenpol, einer Elektrodenzuleitung und einer diese umgebenden Isolationshülle gegenüber einem insbesondere durch Körperflüssigkeit gebildeten Elektrolyten sowie eine Gegenelektrode zu der Elektrodenleitung. Die Erfindung bezieht sich ferner auf ein Verfahren zur Überwachung der Isolation einer Elektrodenleitung eines solchen implantierbaren medizinischen Gerätes.

Zum Hintergrund der Erfindung ist festzuhalten, dass bei solchen implantierbaren medizinischen Geräten eine besondere Problematik darin besteht, dass bei den Isolationshüllen von Elektrodenzuleitungen Dichtigkeitsprobleme beispielsweise aufgrund von Beschädigungen der Isolationshülle oder aufgrund von Spalten und Lücken bei Fugen zwischen verschiedenen Isolationshüllenabschnitten auftreten können. Solche Isolationsdefekte können unter anderem Verfälschungen bei den von der Elektrodenleitung erfassten kardiologischen Signale etwa im Falle eines Herzschrittmachers hervorrufen, wodurch dessen Funktion beeinträchtigt werden kann. Eine zuverlässige Erkennung eines Isolationsdefektes an einer implantierten Elektrodenleitung ist also von großer Wichtigkeit für die Funktionsfähigkeit eines solchen implantierbaren medizinischen Gerätes.

Bekannte Vorrichtungen und Verfahren für die Elektrodenfehlererkennung bedienen sich derzeit in Messung und Auswertung verschiedener Parameter, wie beispielsweise Elektrodenimpedanzen, Signalamplituden, Störsignalerkennung, Reizschwellen, Frequenzanalysen, diverse Plausibilitätsprüfungen und so weiter. Aus dem vielschichtigen Stand der Technik soll als Beispiel nur die US 2011/0160808 A1 herausgegriffen werden, bei der eine Elektrodenfehlererkennung auf der Basis einer Impedanzmessung stattfindet. Als alternative Kontrollmethode wird in dieser Druckschrift ferner offenbart, dass zwischen zwei Elektroden ein Stromimpuls eingespeist und ein sich daraus entwickelndes Antwortsignal in Form einer zwischen den Elektrodenleitungen auftretenden Spannung gemessen werden.

Die Erfahrungen im Umgang mit solchen bekannten Methoden zur Elektrodenfehlererkennung haben jedoch gezeigt, dass diese oft eine zu geringe Sensitivität und Spezifität zeigen. So können Isolationsdefekte häufig nicht ausreichend zuverlässig durch eine Impedanzmessung festgestellt werden, da die Impedanz der eigentlichen Elektrodenpole weit unterhalb der Impedanz eines Isolationsdefektes liegt, so dass der Isolationsdefekt in der Impedanzprüfung nur einen unbedeutenden Impedanzabfall verursacht. Ähnliches gilt für die anderen oben erwähnten Verfahren.

Der Erfindung liegt die Aufgabe zugrunde, ein implantierbares medizinisches Gerät anzugeben, bei dem ein Isolationsfehler im Bereich der implantierten Elektrodenleitung zuverlässig und frühzeitig erkannt werden kann.

Diese Aufgabe wird in gerätetechnischer Hinsicht dadurch gelöst, dass für die Elektrodenzuleitung und den Elektrodenpol bezogen auf die elektrochemische Spannungsreihe unterschiedliche Materialien verwendet werden. Ferner ist eine Isolationsprüfeinrichtung mit einem zwischen dem Elektrodenpol und einer Gegenelektrode geschalteten Gleichspannungsdetektor vorgesehen, um eine elektrochemische Spannung zu erkennen, die bei einem Isolationsfehler der Isolationshülle aufgrund des fehlerhaften Kontaktes zwischen dem Elektrolyten und der Elektrodenzuleitung entsteht.

In verfahrenstechnischer Hinsicht wird die erfindungsgemäße Aufgabe durch das Bereitstellen einer Isolationsprüfeinrichtung mit einem zwischen Elektrodenpol und Gegenelektrode geschalteten Gleichspannungsdetektor, durch Messen der Spannung zwischen dem Elektrodenpol und der Gegenelektrode und durch eine Auswertung der gemessenen Spannung zur Erkennung einer bei einem Isolationsfehler der Isolationshülle aufgrund des Kontaktes zwischen dem Elektrolyten und der Elektrodenzuleitung entstehenden elektrochemischen Spannung gelöst.

Erkennbar beruht die Erfindung auf einem denkbar einfachen physikalisch-chemischen Effekt, der bei intakter Isolationshülle nicht in Erscheinung tritt und damit die eigentlichen Messfunktionen des implantierbaren medizinischen Gerätes nicht beeinflusst. Erst mit Auftreten eines solchen Isolationsfehlers, mit dem die Funktionsfähigkeit des Gerätes ohnehin beeinträchtigt ist, wird mit Hilfe der Isolationsprüfeinrichtung und dem entsprechenden Prüfverfahren eine signifikante Spannungsänderung ausgewertet und zur Fehlererkennung herangezogen.

Gemäß einer bevorzugten Weiterbildung der Erfindung kann die Gegenelektrode der Isolationsprüfeinrichtung durch einen elektrisch leitfähigen Gehäuseabschnitt des implantierbaren medizinischen Gerätes selbst gebildet sein. Dies stellt eine konstruktiv besonders einfache Realisierung des Gegenpols für die Isolationsprüfeinrichtung dar.

Als bevorzugte Materialpaarungen für die Materialauswahl von Elektrodenzuleitung und Elektrodenpol können beispielsweise folgende Werkstoffkombinationen genannt werden: Gold, Platin, Silber oder Kupfer einerseits und Titan, Aluminium, Chrom, MP35N andererseits.
Von Vorteil ist schließlich eine mit dem Gleichspannungsdetektor gekoppelte Signalisierungseinrichtung, mit der ein Isolationsfehler zur Anzeige gebracht werden kann. Eine solche Signalisierungseinrichtung kann beispielsweise durch eine optische Warnleuchte oder einen akustischen Signalgeber gebildet sein, die bei Erkennung einer einen Isolationsfehler indizierenden elektrochemischen Spannung ein Warnsignal abgeben.

Weitere Merkmale, Einzelheiten und Vorteile der Erfindung ergeben sich aus der nachfolgenden Beschreibung eines Ausführungsbeispiels anhand der beigefügten Zeichnungen. Es zeigen:

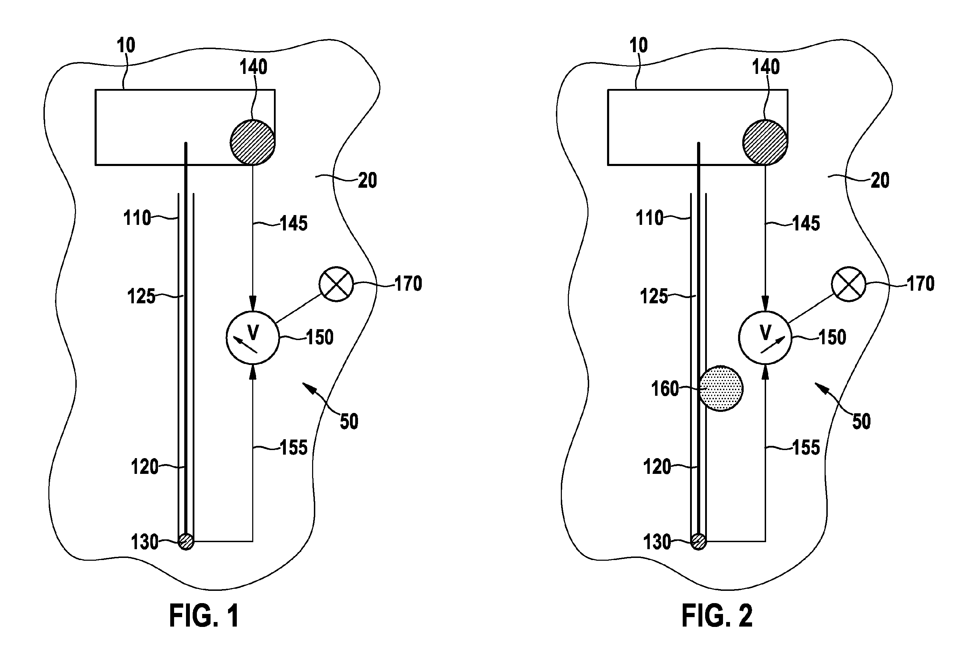

- Fig. 1 und 2: höchst schematische Darstellungen eines implantieren medizinischen Gerätes mit einer Isolationsprüfeinrichtung im ordnungsgemäßen Zustand der Isolationshülle und bei einem Isolationsfehler.

Die Zeichnungen stellen ein aktives elektronisches Implantat beispielsweise in Form eines Herzschrittmachers 10 dar, der in den menschlichen Körper 20 implantiert ist. Der Herzschrittmacher 10 umfasst eine Elektrodenleitung 110, die beispielsweise in den rechten Ventrikel des Herzens eingeführt und dort mit ihrem Elektrodenpol 130 verankert sein kann. Die elektrische Verbindung zwischen dem Elektrodenpol 130 und dem Herzschrittmacher 10 wird über die Elektrodenzuleitung 120 realisiert, die von einer Isolationshülle 125 auf ihrer Länge umgeben ist.

Die Elektrodenzuleitung 120 und der Elektrodenpol 130 bestehen aus zwei unterschiedlichen Metallen, wie beispielsweise Kupfer und Gold, die in der elektrochemischen Spannungsreihe deutlich unterschiedliche Standardpotenziale aufweisen. Bei intakter Isolationshülle 125 wirkt sich diese Materialpaarung auf die über den Elektrodenpol 130 gemessenen Reizpotenziale des Herzens nicht aus.

Zur Überwachung eines intakten Zustandes der Isolationshülle 125 ist eine als Ganzes mit 50 bezeichnete Isolationsprüfeinrichtung vorgesehen. Diese umfasst einen Gleichspannungsdetektor 150 in Form eines Spannungsmessgerätes, der zwischen dem Elektrodenpol 130 und einem Gegenpol 140 über entsprechende Verbindungsleitungen 145, 155 geschaltet ist eine zwischen Elektrodenpol 130 und Gegenpol 140 entstehende Spannung V detektiert. Eine solche Spannung V tritt aufgrund der Materialpaarung von Elektrodenzuleitung 120 und Elektrodenpol 130 dann signifikant auf, wenn die Isolationshülle 125 defekt ist und somit einen in Fig. 2 angedeuteten Kontakt zwischen der Elektrodenzuleitung 120 und dem durch die im Körper 20 vorhandene Körperflüssigkeit gebildeten Elektrolyten 160 zulässt. Die dadurch entstehende elektrochemische Spannung wird vom Gleichspannungsdetektor 150 erfasst und kann über die Signalisierungseinrichtung 170 dem Nutzer des medizinischen Gerätes 10 angezeigt werden. Insgesamt wird ein Defekt der Isolationshülle 125 der Elektrodenleitung 110 also frühzeitig und sehr spezifisch erkannt.

## Patentansprüche

1. Implantierbares medizinisches Gerät, insbesondere aktives elektronisches Implantat, umfassend
- mindestens eine Elektrodenleitung (110) mit einem Elektrodenpol (130), einer Elektrodenzuleitung (120) und einer diese umgebenden Isolationshülle (125) gegenüber einem insbesondere durch Körperflüssigkeit gebildeten Elektrolyten (160), und
- eine Gegenelektrode (140) zu der Elektrodenleitung (110),
**gekennzeichnet durch**
- bezogen auf die elektrochemische Spannungsreihe unterschiedliche Materialien von Elektrodenzuleitung (120) und Elektrodenpol (130), und
- eine Isolationsprüfeinrichtung (50) mit einem zwischen Elektrodenpol (130) und Gegenelektrode (140) geschalteten Gleichspannungsdetektor (150) zur Erkennung einer bei einem Isolationsfehler der Isolationshülle (125) auf Grund des Kontaktes zwischen dem Elektrolyten (160) und der Elektrodenzuleitung (120) entstehenden elektrochemischen Spannung (V).

2. Gerät nach Anspruch 1, **dadurch gekennzeichnet, dass** die Gegenelektrode (140) von einem elektrisch leitfähigen Gehäuseabschnitt des Gerätes gebildet ist.

3. Gerät nach Anspruch 1 oder 2, **dadurch gekennzeichnet, dass** die Materialien für die Elektrodenzuleitung (120) und den Elektrodenpol (130) ausgewählt sind aus folgenden Materialpaarungen: wie Gold, Platin, Silber oder Kupfer einerseits und Titan, Aluminium, Chrom, MP35N andererseits.

4. Gerät nach einem der vorgenannten Ansprüche, **dadurch gekennzeichnet, dass** eine mit dem Gleichspannungsdetektor (150) gekoppelte Signalisierungseinrichtung (170) zur Anzeige eines Isolationsfehlers vorgesehen ist.

5. Verfahren zur Überwachung der Isolation einer Elektrodenleitung (110) eines implantierbaren medizinischen Gerätes (10), welches Gerät (10) umfasst
- mindestens eine Elektrodenleitung (110) mit einem Elektrodenpol (130), einer Elektrodenzuleitung (120) und einer diese umgebenden Isolationshülle (125) gegenüber einem insbesondere durch Körperflüssigkeit gebildeten Elektrolyten (160), und
- eine Gegenelektrode (140) zu der Elektrodenleitung (110),
**gekennzeichnet durch** folgende Verfahrensschritte:
- Bereitstellen einer Isolationsprüfeinrichtung (50) mit einem zwischen Elektrodenpol (130) und Gegenelektrode (140) geschalteten Gleichspannungsdetektor (150)
- Messung der Spannung (V) zwischen Elektrodenpol (130) und Gegenelektrode (140),
- Auswertung der gemessenen Spannung (V) zur Erkennung einer bei einem Isolationsfehler der Isolationshülle (125) aufgrund des Kontaktes zwischen dem Elektrolyten (160) und der Elektrodenzuleitung (120) entstehenden elektrochemischen Spannung (V).

6. Verfahren nach Anspruch 5, **dadurch gekennzeichnet, dass** bei Erkennung einer einen Isolationsfehler indizierenden Spannung (V) ein Warnsignal ausgegeben wird.
